# EUROPEAN PATENT APPLICATION

(11) **EP 4 272 734 A1**
(43) Date of publication of application: **08.11.2023**
(21) Application number: 21915886.2
(22) Date of filing: 30.12.2021
(51) Int. Cl.: A61K 9/20, A61K 33/08, A61K 33/10, A61K 33/04, A61P 1/10, A61K 31/80

(54) **ORAL SOLID FORMULATION FOR COLON CLEANSING**

(30) Priority: 31.12.2020 KR 20200189688; 31.12.2020 KR 20200189689
(71) Applicant: Taejoon Pharmaceutical Co., Ltd., Seoul 04401 (KR)
(72) Inventor: LEE, Joon Youb, Seoul 04401 (KR); GU, Wan, Seongnam-si Gyeonggi-do 13611 (KR); JANG, Woo Young, Suwon-si Gyeonggi-do 16380 (KR); KIM, Young Un, Suwon-si Gyeonggi-do 16434 (KR); KIM, Yong Jun, Suwon-si Gyeonggi-do 16712 (KR)
(74) Representative: Mewburn Ellis LLP
(86) International application number: PCT/KR2021/020327
(87) International publication number: WO 2022/146089

(57) **Abstract**

The present invention relates to an oral solid formulation for colon cleansing, wherein the oral solid formulation minimizes side effects, has excellent dosing convenience and medication compliance, enables a pharmaceutical effect to be exhibited quickly, and has excellent colon cleansing ability.

## Description

### Technical Field

The present invention relates to an oral solid formulation for colon cleansing.

### Background

Colon cancer is one of the most common cancers worldwide, and an incidence thereof is steadily increasing. Colon cancer does not show any special symptoms, making it difficult to detect the same early, and colonoscopy is essential to detect and diagnose colon cancer early.

For an accurate examination and disease diagnosis in colonoscopy, whether or not the colon is washed and a degree of colon cleansing are very important, and if the colon is not cleanly washed, there may be problems in which the diagnosis is not possible or the accuracy of the examination deteriorates. In some cases, the colon cleansing agent may be taken again to perform a re-examination.

In particular, for successful colonoscopy and diagnosis of disease, it is important to take all of the drug solution in accordance with the predetermined usage and dose of the colon cleansing agent. The colon cleansing agents are often prescribed without a face-to-face contact between doctor and patient, and thus the patient often takes the agents on his/her own.

A representative colon cleansing agent having sulfate as an active ingredient includes Suprep Solution consisting of 35 g of anhydrous sodium sulfate, 6.26 g of potassium sulfate, and 3.2 g of anhydrous magnesium sulfate.

However, in the case of liquid for colon cleansing, an amount of the medicinal fluid to be taken is too large or has a bitter taste, and thus the patient often fails to take all the drug solution. In addition, there was a problem in which the patient complains of side effects such as nausea, vomiting or the like after taking the medicine. In order to solve this problem, a tablet-type colon cleansing agent has been developed, but for colon cleansing, about at least 28 tablets having a weight of about 1.6-1.7 g need to be taken. Thus, it is still difficult for the patient to take the tablets because the patient has to swallow bulky tablets, and there remain problems in which the patient complains of gastrointestinal disorders such as nausea, sickness, and abdominal pain after taking the tablets. In addition, bulky tablets may not completely dissolve in the gastrointestinal tract and the residues thereof may remain therein, thus causing gastrointestinal disorders such as rubefaction, etc. The residues may degrade accuracy of endoscopy by obstructing the vision during the gastroscope and colonoscopy, and make it difficult to detect lesions during the endoscopy due to insufficient colon cleansing ability.

Thus, there is a demand for a colon cleansing formulation with improved dosing convenience and medication compliance while showing excellent cleansing ability.

### Detailed Description of the Invention

### Technical Problem

An object of the present invention is to provide an oral solid formulation for colon cleansing.

### Technical Solution

The embodiments of the present invention are described below, but are provided only for the purpose of illustrating the present invention, and the present invention is not limited thereto. Each of the descriptions and embodiments disclosed in the present invention below may be applied to each of other descriptions and embodiments. In other words, all the combinations of various elements disclosed in the present invention fall within the scope of the present invention.

In addition, singular forms may include plural forms and also vice versa unless particularly defined by context of the present specification.

The present invention may provide an oral solid formulation including a purgative component as an active ingredient.

The oral solid formulation of the present invention may be for colon cleansing. In other words, the oral solid formulation of the present invention may be a colon cleansing agent.

The oral solid formulation may include a unit solid formulation, and the unit solid formulation of the oral solid formulation may have a weight of about 0.5 g or less. In addition, the unit solid formulation of the oral solid formulation may have a weight of about 0.05 g or more.

In the present invention, the term "unit solid formulation" may refer to one (singular) solid formulation constituting a solid formulation, and the term "solid formulation" may refer to a group including a plurality of unit solid formulations.

In the present invention, the weight of the unit solid formulation may refer to a weight per unit solid formulation (unit). In other words, the solid formulation of the present invention may include the unit solid formulations having a weight per unit solid formulation (unit) of about 0.5 g or less. In addition, the weight per unit solid formulation (unit) may be about 0.05 g or more.

The solid formulation may include a plurality of unit solid formulations. In embodiments of the present invention, the solid formulation may include about 70 to about 2000 unit solid formulations. Specifically, the solid formulation may include about 70 or more, about 71 or more, about 72 or more, about 80 or more, about 86 or more, about 89 or more, about 90 or more, about 100 or more, about 107 or more, about 110 or more, about 120 or more, about 125 or more, about 129 or more, about 130 or more, about 134 or more, about 140 or more, about 143 or more, about 150 or more, about 160 or more, about 161 or more, about 170 or more, about 172 or more, about 180 or more, about 188 or more, about 190 or more, about 200 or more, about 210 or more, about 215 or more, about 220 or more, about 230 or more, about 240 or more, about 250 or more, about 260 or more, about 270 or more, about 280 or more, about 286 or more, about 290 or more, about 300 or more, about 322 or more, about 344 or more, about 350 or more, about 358 or more, about 387 or more, or about 400 or more unit solid formulations, and may include about 2000 or less, about 1500 or less, about 1434 or less, about 1200 or less, about 1100 or less, about 1075 or less, about 1000 or less, about 968 or less, about 900 or less, about 860 or less, about 800 or less, about 753 or less, about 700 or less, about 645 or less, about 600 or less, about 592, about 500 or less, or about 400 or less unit solid formulations.

In embodiments of the present invention, the unit solid formulation may have a weight of about 0.05 g or more, about 0.1 g or more, about 0.15 g or more, about 0.2 g or more, about 0.25 g or more, and may have a weight of about 0.5 g or less, less than about 0.5 g, about 0.4 g or less, about 0.3 g or less, about 0.25 g or less, about 0.2 g or less.

In the embodiments of the present invention, the unit solid formulation may have a weight of about 0.05 g to about 0.5 g, about 0.1 g to about 0.5 g, about 0.1 g or more and less than about 0.5 g, about 0.1 g to about 0.4 g, about 0.05 g to about 0.4 g, about 0.05 g to about 0.3 g, about 0.1 g to about 0.3 g, and more specifically about 0.1 g to about 0.2 g, or about 0.15 g to about 0.2 g.

The solid formulation including the purgative component of the present invention may include the unit solid formulation having a weight of about 0.5 g or less, specifically less than about 0.5 g, about 0.4 g or less, and more specifically about 0.3 g or less, thereby minimizing side effects, improving dosing convenience, enabling a pharmaceutical effect to be quickly exhibited, and exhibiting excellent colon cleansing ability. In the case of the solid formulation including the purgative component, if the unit solid formulation has a weight higher than the above weight, dysphagia (swallowing disorder), gastrointestinal disorders such as nausea, sickness, vomiting or feeling like vomiting, and/or side effects such as hyperemia, hemorrhage, erosion, edema, and the like of the stomach or large intestine may be remarkably increased when the solid formulation is taken, and thus it is possible to greatly reduce the dosing convenience and medication compliance. In addition, a disintegration rate may be greatly reduced, colon cleansing ability may be reduced, and formulation residues may remain in the body, thus causing blurring of the field of vision. In other words, there may be a remarkable difference in effects in terms of safety (side effects), dosing convenience, medication compliance, colon cleansing ability, disintegration, occurrence of formulation residues and the like between in and out of the weight range of the unit solid formulation of the solid formulation including the purgative component as above.

The purgative component may refer to a component used for the purpose of excreting the contents of the intestine, and the purgative component may include at least one selected from the group consisting of polyethylene glycol, ascorbic acid, a salt of ascorbic acid, sorbitol, mannitol, citric acid, a salt of citric acid, magnesium salt, carboxymethyl cellulose, a salt of carboxymethyl cellulose, lactulose, and sulfate.

The salt may be used without limitation, as long as the salt is any pharmaceutically acceptable salt, but may be, for example, sodium, potassium, magnesium, etc. Specifically, the salt of ascorbic acid may be sodium ascorbate, potassium ascorbate, magnesium ascorbate, etc. In addition, the salt of citric acid may be sodium citrate, potassium citrate, magnesium citrate, etc. Furthermore, the salt of carboxymethyl cellulose may be sodium carboxymethyl cellulose.

In embodiments of the present invention, magnesium salt may include at least one selected from the group consisting of magnesium oxide, magnesium oxide heavy, magnesium hydroxide, magnesium carbonate, magnesium carbonate heavy, and magnesium sulfate.

In embodiments of the present invention, the sulfate may include at least one selected from the group consisting of sodium sulfate, potassium sulfate, and magnesium sulfate. In embodiments of the present invention, the sulfate may be sodium sulfate and potassium sulfate. In other embodiments, the sulfate may be sodium sulfate and magnesium sulfate. In still other embodiments, the sulfate may be a mixture of sodium sulfate, potassium sulfate, and magnesium sulfate. In addition, the sulfate may be an anhydride or a hydrate, and preferably the sodium sulfate or the magnesium sulfate may be an anhydride.

The solid formulation may include the purgative component in an amount of about 20 g or more. Specifically, the solid formulation may include the purgative component in an amount of about 25 g or more, about 30 g or more, about 35 g or more, but is not necessarily limited thereto. The solid formulation may include the purgative component in an amount of about 500 g or less. Specifically, the solid formulation may include the purgative component in an amount of about 465 g or less, about 420 g or less, about 372 g or less, about 300 g or less, about 279 g or less, about 270 g or less, about 260 g or less, about 240 g or less, about 230 g or less, about 200 g or less, about 186 g or less. More specifically, the solid formulation may include the purgative component in an amount of about 150 g or less, about 140 g or less, about 130 g or less, about 120 g or less, about 110 g or less, about 100 g or less, about 93 g or less, about 90 g or less, about 80 g or less, about 70 g or less, about 60 g or less, about 55 g or less, but is not necessarily limited thereto.

In embodiments of the present invention, the solid formulation may include the purgative component in a total amount of about 20 to about 500 g. Specifically, the solid formulation may include the purgative component in a total amount of about 20 to about 420 g, about 20 to about 300 g, about 20 to about 200 g, or about 20 to about 150 g. More specifically, the solid formulation may include the purgative component in an amount of about 20 to about 100 g, about 20 to about 90 g, about 20 to about 80 g, about 25 to about 100 g, about 25 to about 90 g, about 25 to about 80 g, about 25 to about 70 g, about 25 to about 60 g, or about 25 to about 55 g, but is not necessarily limited thereto.

In embodiments of the present invention, the solid formulation may include the sulfate in a total amount of about 0.1 g or more, about 5 g or more, about 10 g or more, and may include the sulfate in a total amount of about 60 g or less, about 50 g or less, about 45 g or less, about 40 g or less, but is not limited thereto. In embodiments of the present invention, the solid formulation may include the sulfate in a total amount of about 0.1 to about 60 g. Specifically, the solid formulation may include the sulfate in a total amount of about 5 to about 50 g. More specifically, the solid formulation may include the sulfate in an amount of about 5 to about 45 g, or about 10 to about 40 g, but is not necessarily limited thereto.

In embodiments of the present invention, the solid formulation may include polyethylene glycol in a total amount of about 0.1 g or more, about 5 g or more, about 10 g or more, and may include polyethylene glycol in a total amount of about 450 g or less, about 300 g or less, about 250 g or less, about 200 g or less, about 100 g or less, about 80 g or less, about 50 g or less, about 40 g or less, but is not necessarily limited thereto. In embodiments of the present invention, the solid formulation may include the polyethylene glycol in a total amount of about 0.1 to about 450 g. Specifically, the solid formulation may include polyethylene glycol in a total amount of about 0.1 to about 300 g, or about 5 to about 250 g. More specifically, the solid formulation may include polyethylene glycol in a total amount of about 10 to about 200 g, about 10 to about 100 g, about 10 to about 80 g, about 10 to about 50 g, about 10 to about 40 g, but is not necessarily limited thereto.

In embodiments of the present invention, the solid formulation may include the ascorbic acid and/or the salt thereof in a total amount of about 0.1 g or more, about 5 g or more, about 10 g or more, and may include the ascorbic acid and/or the salt thereof in a total amount of about 150 g or less, about 100 g or less, about 50 g or less, about 30 g or less, but is not necessarily limited thereto. In embodiments of the present invention, the solid formulation may include the ascorbic acid and/or the salt thereof in a total amount of about 0.1 to about 150 g, specifically about 5 to about 100 g, and more specifically about 5 to about 50 g, about 10 to about 30 g, but is not necessarily limited thereto.

In embodiments of the present invention, the solid formulation may include the sorbitol and/or mannitol in a total amount of about 0.1 g or more, about 5 g or more, about 10 g or more, and may include the sorbitol and/or mannitol in a total amount of about 150 g or less, about 100 g or less, about 50 g or less, but is not necessarily limited thereto. In embodiments of the present invention, the solid formulation may include the sorbitol and/or mannitol in a total amount of about 0.1 to about 150 g, specifically about 5 to about 100 g, and more specifically about 5 to about 50 g, but is not necessarily limited thereto.

In embodiments of the present invention, the solid formulation may include the citric acid and/or the salt thereof in a total amount of about 0.1 g or more, about 1 g or more, about 10 g or more, and may include the citric acid and/or the salt thereof in a total amount of about 100 g or less, about 50 g or less, about 30 g or less, but is not limited thereto. In embodiments of the present invention, the solid formulation may include the citric acid and/or the salt thereof in a total amount of about 0.1 to about 100 g, specifically about 1 to about 50 g, and more specifically about 10 to about 30 g, but is not limited thereto.

In embodiments of the present invention, the solid formulation may include the magnesium salt in a total amount of about 0.1 g or more, about 1 g or more, and may include the magnesium salt in a total amount of about 30 g or less, about 10 g or less, but is not limited thereto. In embodiments of the present invention, the solid formulation may include the magnesium salt in a total amount of about 0.1 to about 30 g, specifically about 1 to about 30 g, and more specifically about 1 to about 10 g, but is not limited thereto.

In embodiments of the present invention, the solid formulation may include the carboxymethyl cellulose and/or the salt thereof in a total amount of about 0.1 g or more, about 0.2 g or more, and may include the carboxymethyl cellulose and/or the salt thereof in a total amount of about 20 g or less, about 12 g or less, about 8 g or less, about 6 g or less, but is not limited thereto. In embodiments of the present invention, the solid formulation may include the carboxymethyl cellulose and/or the salt thereof in a total amount of about 0.1 to about 20 g, specifically about 0.2 to about 6 g, but is not limited thereto.

In embodiments of the present invention, the solid formulation may include the lactulose in a total amount of about 1 g or more, about 2 g or more, about 5 g or more, and may include the lactulose in a total amount of about 100 g or less, about 50 g or less, about 40 g or less, but is not limited thereto. In embodiments of the present invention, the solid formulation may include the lactulose in a total amount of about 1 to about 100 g, specifically about 2 to about 50 g, and more specifically about 5 to about 40 g, but is not limited thereto.

In the solid formulation, the content of the purgative component may mean a total content of the purgative components included in a plurality of unit solid formulations included in the solid formulation. The solid formulation including the purgative component may include the content of the purgative component as above and thus exhibit excellent colon cleansing ability while minimizing side effects.

In embodiments of the present invention, the unit solid formulation of the present invention may include the purgative component in a total amount of about 20 mg or more, about 25 mg or more, about 30 mg or more, about 35 mg or more, about 40 mg or more, about 45 mg or more, and may include the purgative component in a total amount of about 500 mg or less, less than about 500 mg, about 450 mg or less, about 400 mg or less, about 350 mg or less, specifically about 300mg or less, about 295 mg or less, about 290 mg or less, about 285 mg or less, about 280 mg or less, about 275 mg or less, about 270 mg or less, but is not limited thereto.

In embodiments of the invention, the unit solid formulation may include the purgative component in an amount of about 20 mg to about 500 mg. In embodiments of the invention, the unit solid formulation may include the purgative component in an amount of about 20 mg or more and less than 500 mg. Specifically, the unit solid formulation may include the purgative component in a total amount of about 20 mg to about 300 mg, about 25 mg to about 295 mg, and more specifically the unit solid formulation may include the purgative component in an amount of about 29 mg to about 290 mg, about 40 mg to about 290 mg, about 45 mg to about 290 mg, about 40 mg to about 295 mg, about 45 mg to about 295 mg, about 40 mg to about 285 mg, or about 45 mg to about 285 mg, but is not necessarily limited thereto.

The unit solid formulation of the present invention may include the purgative component in an amount of about 40% or more, about 50% or more, about 60% or more, about 70% or more, about 80% or more, about 90% or more, about 95% or more, about 96% or more, about 97% or more, about 98% or more, about 99% or more, about 100% or about 100% or less of a weight of the unit solid formulation, but is not limited thereto.

The unit solid formulation including the purgative component may include the same content of the purgative component as described above, thereby exhibiting excellent colon cleansing ability while minimizing side effects, and thus the unit solid formulation may have excellent physical properties when prepared.

The solid formulation may further include simethicone. The simethicone may be included in the solid formulation as an active ingredient.

In embodiments of the present invention, the solid formulation may include simethicone in a total amount of about 0.01 g to about 1.2 g. Specifically, the solid formulation may include simethicone in a total amount of about 0.01 g or more, about 0.04 g or more, about 0.1 g or more, about 0.2 g or more, about 0.3 g or more, or about 0.4 g or more, and may include simethicone in an amount of about 1.2 g or less, about 1 g or less, about 0.8 g or less, about 0.6 g or less, about 0.5 g or less.

In embodiments of the invention, the unit solid formulation may include simethicone in an amount of about 0.005 mg to about 17.14 mg. Specifically, the unit solid formulation may include simethicone in an amount of about 0.04 mg to about 17.14 mg, about 0.04 mg to about 14.3 mg. Specifically, the unit solid formulation may include simethicone in an amount of about 1 to about 2 mg.

The solid formulation may further include an additional active ingredient. For example, the solid formulation may further include a stimulant laxative. For example, the solid formulation may further include at least one of components such as bisacodyl, castor oil, senna, bisoxatin, docusate, picosulfate, linaclotide, lubiprostone, dietary fiber, a bulk forming laxative (plantago seed, calcium polycarbophil, methylcellulose, wheat bran, etc.), anthraquinone, diphenylmethane, probiotics, and the like.

In addition, the present invention may provide an oral solid formulation including sulfate as an active ingredient.

The oral solid formulation of the present invention may be for colon cleansing. In other words, the oral solid formulation of the present invention may be a colon cleansing agent.

The oral solid formulation may include a unit solid formulation, and the unit solid formulation of the oral solid formulation may have a weight of about 0.3 g or less. In addition, the unit solid formulation of the oral solid formulation may have a weight of about 0.05 g or more.

In the present invention, the term "unit solid formulation" may refer to one (singular) solid formulation constituting a solid formulation, and the term "solid formulation" may refer to a group including a plurality of unit solid formulations.

In the present invention, the weight of the unit solid formulation may refer to a weight per unit solid formulation (unit). In other words, the solid formulation including sulfate as the active ingredient of the present invention may include the unit solid formulations each (unit) of which has a weight of about 0.3 g or less. In addition, each (unit) of the unit solid formulation of the solid formulation including sulfate as the active ingredient may have a weight of about 0.05 g or more.

The solid formulation including sulfate as the active ingredient may include a plurality of unit solid formulations including sulfate as an active ingredient. In embodiments of the present invention, the solid formulation including sulfate as the active ingredient may include about 80 to about 1200 unit solid formulations including sulfate as the active ingredient. Specifically, the solid formulation may include about 80 or more, about 90 or more, about 100 or more, about 110 or more, about 120 or more, about 130 or more, about 140 or more, about 150 or more, about 160 or more, about 170 or more, about 180 or more, about 190 or more, about 200 or more, about 210 or more, about 220 or more, about 230 or more, about 240 or more, about 250 or more, about 260 or more, about 270 or more, about 280 or more, about 290 or more, about 300 or more, about 320 or more, or about 350 or more unit solid formulations, and may include about 1200 or less, about 1100 or less, about 1000 or less, about 900 or less, about 800 or less, about 700 or less, about 600 or less, or about 500 or less unit solid formulations.

In embodiments of the present invention, the unit solid formulation including sulfate as the active ingredient may have a weight of about 0.05 g or more, about 0.1 g or more, about 0.15 g or more, about 0.2 g or more, about 0.25 g or more and may have a weight of about 0.3 g or less, about 0.25 g or less, about 0.2 g or less.

In embodiments of the present invention, the unit solid formulation including sulfate as the active ingredient may have a weight of about 0.05 g to about 0.3 g, about 0.1 g to about 0.3 g, more specifically about 0.1 g to about 0.2 g, or about 0.15 g to about 0.2 g, but is not limited thereto.

The solid formulation including sulfate as the active ingredient of the present invention may have the unit solid formulation having a weight of about 0.3 g or less, thereby minimizing dysphagia (swallowing disorder), gastrointestinal disorders such as nausea, sickness, vomiting or feeling like vomiting, or side effects such as hyperemia, hemorrhage, erosion, edema, and the like of the stomach or large intestine when the solid formulation is taken, improving dosing convenience, enabling a pharmaceutical effect to be quickly exhibited, and exhibiting excellent colon cleansing ability. In addition, no formulation residues may remain in the gastrointestinal tract, and a clear visual field may be secured during colonoscopy. However, in the case of a solid formulation including sulfate as the active ingredient, when the unit solid formulation has a weight greater than about 0.3 g, there may be a remarkable difference in gastrointestinal disorders, and/or side effects and the like, and dosing convenience and medication compliance may be greatly reduced. In addition, a disintegration rate may be greatly reduced, colon cleansing ability may be reduced, and formulation residues may remain in the body, thus causing blurring of the field of vision. In other words, there may be a remarkable difference in effects in terms of safety (side effects), dosing convenience, medication compliance, colon cleansing ability, disintegration, occurrence of formulation residues and the like between in and out of the weight range of the unit solid formulation of the solid formulation including the sulfate as above.

In this case, in embodiments of the present invention, the solid formulation including sulfate as the active ingredient and the unit solid formulation thereof may be a solid formulation in which an active ingredient is composed of (consists of) sulfate. Alternatively, the solid formulation may be a solid formulation in which active ingredients are composed of (consist of) sulfate and simethicone.

In the solid formulation, the sulfate may include at least one selected from the group consisting of sodium sulfate, potassium sulfate, and magnesium sulfate. In embodiments of the present invention, the sulfate may include sodium sulfate. In other embodiments, the sulfate may include potassium sulfate. In still other embodiments, the sulfate may include magnesium sulfate. In embodiments of the present invention, the sulfate may be sodium sulfate and potassium sulfate. In other embodiments, the sulfate may be sodium sulfate and magnesium sulfate. In still other embodiments, the sulfate may be a mixture of sodium sulfate, potassium sulfate, and magnesium sulfate. In addition, the sulfate may be an anhydride or a hydrate, and preferably the sodium sulfate or the magnesium sulfate may be an anhydride.

In embodiments of the present invention, the solid formulation including sulfate as the active ingredient may include the sulfate in a total amount of about 25 g or more. Specifically, the solid formulation may include the sulfate in a total amount of about 30 g or more, about 35 g or more, about 40 g or more, about 45 g or more, but is not necessarily limited thereto. The solid formulation including sulfate as the active ingredient may include the sulfate in a total amount of about 60 g or less. Specifically, the solid formulation may include the sulfate in a total amount of about 55 g or less, about 50 g or less, about 45 g or less, about 40 g or less, but is not necessarily limited thereto. For example, the solid formulation may include the sulfate in a total amount of about 44.46 g.

In embodiments of the present invention, the solid formulation including sulfate as the active ingredient may include the sulfate in a total amount of about 25 to about 60 g. Specifically, the solid formulation may include the sulfate in a total amount of about 25 to about 55 g, about 30 to about 60 g, about 30 to about 55 g, or about 30 to about 50 g. More specifically, the solid formulation may include the sulfate in a total amount of about 40 to about 55 g, about 40 to about 50 g, about 35 to about 50 g, about 35 to about 45 g, about 30 to about 45 g, or about 30 to about 40 g, but is not necessarily limited thereto.

In embodiments of the present invention, when the sulfate includes sodium sulfate, the solid formulation including sulfate as the active ingredient may include sodium sulfate in a total amount of about 10 g to about 60 g. Specifically, the solid formulation may include sodium sulfate in a total amount of about 10 g or more, about 15 g or more, about 20 g or more, about 25 g or more, about 30 g or more, about 33 g or more, or about 35 g or more, and may include sodium sulfate in a total amount of about 60 g or less, about 55 g or less, about 52 g or less, about 50 g or less, about 45 g or less, about 42 g or less, about 40 g or less, or about 36 g or less.

In embodiments of the present invention, when the sulfate includes potassium sulfate, the solid formulation including sulfate as the active ingredient may include potassium sulfate in a total amount of about 0.1 g to about 15 g. Specifically, the solid formulation may include potassium sulfate in a total amount of about 0.1 g or more, about 0.5 g or more, about 1 g or more, about 2 g or more, about 3 g or more, about 4 g or more, or about 5 g or more, or about 6 g or more, and may include potassium sulfate in a total amount of about 15 g or less, about 12 g or less, about 10 g or less, about 9 g or less, about 8 g or less, or about 7 g or less.

In embodiments of the present invention, when the sulfate includes magnesium sulfate, the solid formulation including sulfate as the active ingredient may include magnesium sulfate in a total amount of about 0.1 g to about 8 g. Specifically, the solid formulation may include magnesium sulfate in a total amount of about 0.1 g or more, about 0.5 g or more, about 1 g or more, about 2 g or more, about 3 g or more, or about 4 g or more, and may include magnesium sulfate in a total amount of about 8 g or less, about 7 g or less, or about 6 g or less.

A total content of sulfate in the solid formulation including sulfate as the active ingredient may refer to a total content of sulfate included in a plurality of unit solid formulations included in the solid formulation including sulfate as the active ingredient. The solid formulation including sulfate as the active ingredient may include the content of sulfate as above and thus exhibit excellent colon cleansing ability while minimizing side effects.

The unit solid formulation of the solid formulation including sulfate as the active ingredient of the present invention may include sulfate in an amount of about 20 mg to about 300 mg. Specifically, the unit solid formulation may include sulfate in a total amount of about 20 mg or more, about 25 mg or more, about 30 mg or more, about 35 mg or more, about 40 mg or more, about 45 mg or more, and may include sulfate in a total amount of about 300 mg or less, about 295 mg or less, about 290 mg or less, about 285 mg or less, about 280 mg or less, about 275 mg or less, about 270 mg or less, but is not limited thereto.

In embodiments of the present invention, the unit solid formulation of the solid formulation including sulfate as the active ingredient may include the sulfate in a total amount of about 25 mg to about 295 mg. More specifically, the unit solid formulation may include the sulfate in an amount of about 29 mg to about 290 mg, about 40 mg to about 290 mg, about 45 mg to about 290 mg, about 40 mg to about 295 mg, about 45 mg to about 295 mg, about 40 mg to about 285 mg, or about 45 mg to about 285 mg, but is not necessarily limited thereto.

The unit solid formulation of the solid formulation including sulfate as the active ingredient of the present invention may include the sulfate in an amount of about 6.7% or more, about 9% or more, about 10% or more, about 20% or more, about 30% or more, and more specifically 40% or more, about 50% or more, about 60% or more, about 70% or more, about 80% or more, about 90% or more, about 95% or more, about 96% or more, about 97% or more, about 98% or more, about 99% or more, about 100% or about 100% or less by weight based on a weight of the unit solid formulation, but is not limited thereto.

In embodiments of the present invention, when the sulfate includes sodium sulfate, the unit solid formulation may include sodium sulfate in an amount of about 8 mg to about 300 mg. Specifically, the unit solid formulation may include sodium sulfate in an amount of about 8 mg or more, about 12 mg or more, about 15 mg or more, about 16 mg or more, about 20 mg or more, about 25 mg or more, about 27 mg or more, about 29 mg or more, about 30 mg or more, and may include sodium sulfate in a total amount of about 300 mg or less, about 295 mg or less, about 290 mg or less, about 285 mg or less, about 280 mg or less, about 275 mg or less, about 270 mg or less, but is not limited thereto.

In embodiments of the present invention, the unit solid formulation may include sodium sulfate in an amount of about 8 mg to about 300 mg, about 12 mg to about 300 mg, or about 15 mg to about 300 mg, and more specifically the unit solid formulation may include sodium sulfate in an amount of about 30 to about 270 mg, about 30 to about 250 mg, or about 50 to about 220 mg, and more specifically the unit solid formulation may include sodium sulfate in an amount of about 80 mg to about 170 mg, but is not limited thereto.

In embodiments of the present invention, when the sulfate includes sodium sulfate, the unit solid formulation may include sodium sulfate in an amount of about 2.67% or more, about 5% or more, about 10% or more, specifically about 12% or more, about 16% or more, about 20% or more, about 30% or more, about 40% or more, about 60% or more, about 75% or more, about 80% or more, about 85% or more, about 90% or more, about 95% or more, about 100% or about 100% or less of a weight of the unit solid formulation, but is not limited thereto.

In embodiments of the present invention, when the sulfate includes potassium sulfate, the unit solid formulation may include potassium sulfate in an amount of about 0.08 mg to about 187.5 mg. Specifically, the unit solid formulation may include potassium sulfate in an amount of about 0.08 mg or more, about 0.4 mg or more, about 0.8 mg or more, about 1 mg or more, about 2 mg or more, about 3 mg or more, about 4 mg or more, about 5 mg or more, about 9 mg or more, about 12 mg or more, and the unit solid formulation may include potassium sulfate in an amount of about 187.5 mg or less, about 180 mg or less, about 170 mg or less, about 150 mg or less, about 130 mg or less, about 120 mg or less, about 100 mg or less, about 90 mg or less, about 80 mg or less, about 50 mg or less, about 40 mg or less, about 30 mg or less, but is not limited thereto.

In embodiments of the present invention, the unit solid formulation may include potassium sulfate in an amount of about 2 mg to about 50 mg, and specifically the unit solid formulation may include potassium sulfate in an amount of about 5 mg to about 38 mg, about 9 mg to about 32 mg, or about 12 mg to about 26 mg, but is not limited thereto.

In embodiments of the present invention, when the sulfate includes potassium sulfate, the unit solid formulation may include potassium sulfate in an amount of about 0.01% or more, about 0.02% or more, about 0.03% or more, about 0.05% or more, about 0.067% or more, about 0.08% or more, about 0.1% or more, about 0.3% or more, about 0.4% or more, about 0.5% or more, about 1% or more, about 1.5% or more, about 2% or more, about 3% or more, about 4% or more, about 5% or more, about 6% or more, about 8% or more, about 10% or more, about 20% or more, about 30% or more, about 50% or more, about 60% or about 60% or less by weight based on a weight of the unit solid formulation, but is not limited thereto.

In embodiments of the present invention, when the sulfate includes magnesium sulfate, the unit solid formulation may include magnesium sulfate in an amount of about 0.08 mg to about 100 mg. Specifically, the unit solid formulation may include magnesium sulfate in an amount of about 0.08 mg or more, about 0.4 mg or more, about 0.8 mg or more, about 1 mg or more, about 2 mg or more, about 3 mg or more, about 4 mg or more, about 6 mg or more, about 8 mg or more, about 10 mg or more, about 12 mg or more, and the unit solid formulation may include magnesium sulfate in an amount of about 100 mg or less, about 90 mg or less, about 80 mg or less, about 70 mg or less, about 60 mg or less, about 50 mg or less, about 40 mg or less, about 30 mg or less, about 20 mg or less, about 10 mg or less, but is not limited thereto.

In embodiments of the invention, the unit solid formulation may include magnesium sulfate in an amount of about 1 mg to about 25 mg. Specifically, the unit solid formulation may include magnesium sulfate in an amount of about 1 mg to about 13 mg, about 1 mg to about 12 mg, or about 1 mg to about 10 mg, but is not limited thereto.

In embodiments of the present invention, when the sulfate includes magnesium sulfate, the unit solid formulation may include magnesium sulfate in an amount of about 0.01% or more, about 0.02% or more, about 0.03% or more, about 0.05% or more, about 0.067% or more, about 0.08% or more, about 0.1% or more, about 0.3% or more, about 0.4% or more, about 0.5% or more, about 0.8% or more, about 1% or more, about 1.5% or more, about 2% or more, about 3% or more, about 4% or more, about 5% or more, about 10% or more, about 20% or more, about 30% or more, about 32% or about 32% or less by weight based on a weight of the unit solid formulation, but is not limited thereto.

The unit solid formulation including sulfate as the active ingredient may include the same content of sulfate as described above, thereby exhibiting excellent colon cleansing ability while minimizing side effects, and thus the unit solid formulation may have excellent physical properties when prepared.

The solid formulation including sulfate as the active ingredient may further include simethicone. The simethicone may be included in the solid formulation as an active ingredient.

In embodiments of the present invention, the solid formulation including sulfate as the active ingredient may include simethicone in a total amount of about 0.01 g to about 1.2 g. Specifically, the solid formulation may include simethicone in a total amount of about 0.01 g or more, about 0.04 g or more, about 0.1 g or more, about 0.2 g or more, about 0.3 g or more, or about 0.4 g or more, and may include simethicone in an amount of about 1.2 g or less, about 1 g or less, about 0.8 g or less, about 0.6 g or less, about 0.5 g or less.

In embodiments of the present invention, the unit solid formulation of the solid formulation including sulfate as the active ingredient may include simethicone in an amount of about 0.008 mg to about 15 mg. Specifically, the unit solid formulation may include simethicone in an amount of about 0.008 mg or more, about 0.01 mg or more, about 0.05 mg or more, about 0.1 mg or more, about 0.5 mg or more, about 1 mg or more, and may include simethicone in an amount of about 15 mg or less, about 12.5 mg or less, about 10 mg or less, about 8 mg or less, about 6 mg or less, but is not limited thereto.

In embodiments of the present invention, the unit solid formulation may include simethicone in an amount of about 0.04 mg to about 15 mg, and more specifically the unit solid formulation may include simethicone in an amount of about 1 mg to about 2 mg, but is not limited thereto.

In embodiments of the present invention, the solid formulation and the unit solid formulation, including sulfate as the active ingredient of the present invention, may mean a solid formulation and a unit solid formulation composed of (consisting of) sulfate or sulfate and simethicone as active ingredient. In the solid formulation in which the active ingredient is composed of (consists of) sulfate or sulfate and simethicone, side effects may be minimized, dosing convenience may be improved, and excellent colon cleansing ability may be exhibited in the unit solid formulation having a weight of about 0.3 g or less. However, when the unit solid formulation has a weight more than about 0.3 g, side effects may be remarkably increased, and there may be a remarkable difference in effects in terms of safety (side effects), dosing convenience, medication compliance, colon cleansing ability, disintegration, occurrence of formulation residues and the like.

In the present invention, the term "active ingredient" may have the same meaning as the effective ingredient, and the active ingredient may also be referred to as the effective ingredient.

In the present invention, the term "oral solid formulation" may have the same meaning as a solid formulation for oral administration, and may also be referred to as a solid formulation for oral administration.

The solid formulation of the present invention may further include pharmaceutically acceptable additives.

In the present specification, the pharmaceutically acceptable additive may refer to components which do not impair the effect of the active ingredient. The additive used herein may include, for example, filler, disintegrant, binder, plasticizer, glidant, coating agent, pH adjusting agent, diluent, lubricant, preservative, buffering agent, sweetener, wetting agent, suspending agent, colorant, fragrance, excipient, etc., and any pharmaceutically acceptable ones, which are conventionally used in each dosage form, may be also used.

The additive may be a known additive.

For example, the disintegrant used herein may include crospovidone, guar gum, xanthan gum, sodium starch glycolate, low substituted hydroxypropylcellulose, croscarmellose sodium, microcrystalline cellulose, dextran, mannitol, a mixture thereof or the like, but is not limited thereto.

For example, the binder used herein may include alginic acid, sodium alginate, carbomer, copovidone, starch, polyethylene glycol, polyvinylpyrrolidone, polyvinyl derivative, microcrystalline cellulose, methylcellulose, ethylcellulose, hydroxyethylcellulose, hydroxypropylcellulose, low substituted hydroxypropylcellulose, hydroxypropylmethylcellulose, a mixture thereof or the like, but is not limited thereto.

For example, the glidant used herein may include stearic acid, stearate, stearyl fumarate, lauryl sulfate, stearyl sulfate, vegetable oil, polyethylene glycol, poloxamer, caprylate, a mixture thereof or the like, but is not limited thereto.

For example, the coating agent used herein may include polyvinyl alcohol-polyethylene glycol copolymer, amino methacrylate copolymer, methyl methacrylate copolymer, sucrose, hypromellose, hydroxyethyl cellulose, hydroxyethyl methyl cellulose, carboxymethyl cellulose, hydroxypropyl cellulose, polyethylene glycol, ethyl cellulose, a mixture thereof or the like, but is not limited thereto.

The unit solid formulation of the present invention may have a circular or elliptical shape, but is not necessarily limited thereto.

In embodiments of the present invention, when the unit solid formulation has a circular or elliptical shape, the unit solid formulation may have a diameter or length of about 1 mm to about 20 mm, about 1 mm to about 15 mm, specifically about 1 to about 12 mm, about 1 to about 10 mm, but is not necessarily limited thereto.

In embodiments of the present invention, when the unit solid formulation has a circular or elliptical shape, the unit solid formulation may have a thickness or height of about 1 mm to about 10 mm, specifically about 1 to about 5 mm, but is not necessarily limited thereto.

In the solid formulation of the present invention, the unit solid formulation may have a form of tablet, pellet, capsule, or pill, and the solid formulation may include a unit solid formulation in the form of tablet, pellet, capsule, or pill, or any combination thereof. In embodiments of the present invention, the unit solid formulation may be a tablet, and the solid formulation may include the unit solid formulation in the form of tablet. In addition, the tablet may be a naked or coated tablet.

The oral solid formulation of the present invention may be prepared by an appropriate method according to a specific dosage form thereof. In embodiments of the present invention, when the solid formulation is prepared in a tablet form, the solid formulation may be prepared using a granulation process, a mixing process, and/or a tableting process. The granulation process may be appropriately selected from a dry granulation process, a wet granulation process, and a coating process.

The unit solid formulation of the present invention may be prepared to have a weight of about 0.05 g or more, more specifically about 0.1 g or more, thereby improving production efficiency.

The unit solid formulation of the present invention may be easily prepared and have excellent physical properties as a solid formulation. In addition, the unit solid formulation of the present invention may have the weight as described above, and thus may not get stuck in the esophagus or may not lead to any feeling of irritation caused by foreign matter when taken, and may minimize side effects such as dysphagia (swallowing disorder), gastrointestinal disorders like nausea, sickness, vomiting or feeling like vomiting. In addition, side effects such as hyperemia, hemorrhage, erosion, edema and the like may be minimized in the stomach or large intestine when taken. Thus, the solid formulation of the present invention may have very excellent dosing convenience and medication compliance, thereby facilitating successful completion of dosing, exhibiting an excellent colon cleansing effect, and minimizing side effects from administration. In addition, the solid formulation may quickly disintegrate and dissolve in the stomach after taken, may reduce side effects such as hyperemia, hemorrhage, erosion, edema and the like in the stomach after taken, and may enable a pharmaceutical effect to be quickly exhibited, thereby reducing the time to start and finish discharging stool. Thus, a user's pain caused by defecation may be minimized. Furthermore, the solid formulation may have no formulation residues remaining in the gastrointestinal tract, and may have a clear visual field secured during colonoscopy.

The oral solid formulation of the present invention may be taken in such a way that a total dosage thereof is all taken at one point of time (called non-split administration or split-dose administration on the day) or taken at several points of time by splitting a total dosage of the composition (called split-dose administration or two-day split-dose administration).

As one example of non-split administration (split-dose administration on the day), the oral solid formulation of the present invention may be taken over several hours in the evening of the day before an examination or in the morning of the day of the examination. Specifically, a portion of the total dosage may be taken, and then the rest thereof may be taken in a certain period of time later, for example, in about 0.5 to 3 hours later.

As one example of split-dose administration (or two-day split-dose administration), the oral solid formulation of the present invention may be taken in such a way that a part thereof is taken in the evening of the day before an examination and then the rest thereof is taken in the morning of the day of the examination.

When taking the oral solid formulation, each dosage may be taken within a certain period of time. Specifically, each dosage may be taken within about two hours, about one hour, or about 30 minutes.

The oral solid formulation of the present invention may be taken together with about 1 L or more of water. Specifically, the oral solid formulation may be taken together with about 2 L or more of water. The oral solid formulation of the present invention may be taken together with about 1 to 4 L, about 2 to 3 L of water.

In embodiments of the present invention, there may be provided (1) an oral solid formulation in which the oral solid formulation of the present invention includes sulfate as an active ingredient and each unit solid formulation has a weight of about 0.3 g or less.
(2) In above (1), there may be provided the oral solid formulation in which the solid formulation includes the unit solid formulation each of which has a weight of about 0.05 g or more.
(3) In any one of above (1) and (2), there may be provided the oral solid formulation in which the solid formulation includes the sulfate in an amount of about 25 to about 60 g.
(4) In any one of above (1) to (3), there may be provided the oral solid formulation in which the sulfate includes at least one selected from the group consisting of sodium sulfate, potassium sulfate, and magnesium sulfate.
(5) In any one of above (1) to (4), there may be provided the oral solid formulation in which the sulfate includes sodium sulfate and potassium sulfate.
(6) In any one of above (1) to (5), there may be provided the oral solid formulation in which the sulfate includes sodium sulfate, magnesium sulfate, and potassium sulfate.
(7) In any one of above (1) to (6), there may be provided the oral solid formulation which further includes simethicone.
(8) In any one of above (1) to (7), there may be provided the oral solid formulation which further includes a pharmaceutically acceptable additive.
(9) In any one of above (1) to (8), there may be provided the oral solid formulation in which the solid formulation includes about 80 to about 1200 unit solid formulations.
(10) In any one of above (1) to (9), there may be provided the oral solid formulation in which the unit solid formulation is in the form of tablet, pellet, capsule, or pill.
(11) In any one of above (1) to (10), there may be provided the oral solid formulation in which the solid formulation is for colon cleansing.
(12) In any one of above (1) to (11), there may be provided the oral solid formulation in which the unit solid formulation includes sulfate in an amount of about 20 mg to about 300 mg.
(13) In any one of above (1) to (12), there may be provided the oral solid formulation in which the solid formulation includes simethicone in a total amount of about 0.01 g to about 1.2 g.
(14) In any one of above (1) to (13), there may be provided the oral solid formulation in which the unit solid formulation includes simethicone in an amount of about 0.008 mg to about 15 mg.

In other embodiments of the present invention, there may be provided (i) an oral solid formulation in which the oral solid formulation of the present invention includes a purgative component as an active ingredient and each unit solid formulation has a weight of about 0.5 g or less.
(ii) In above (i), there may be provided the oral solid formulation in which the oral solid formulation includes the unit solid formulation each of which has a weight of about 0.05 g or more.
(iii) In any one of above (i) and (ii), there may be provided the oral solid formulation in which the oral solid formulation includes as the purgative component at least one selected from the group consisting of polyethylene glycol, ascorbic acid, a salt of ascorbic acid, sorbitol, mannitol, citric acid, a salt of citric acid, a magnesium salt, carboxymethyl cellulose, a salt of carboxymethyl cellulose, lactulose, and sulfate.
(iv) In any one of above (i) to (iii), there may be provided the oral solid formulation in which the magnesium salt includes at least one selected from the group consisting of magnesium oxide, magnesium oxide heavy, magnesium hydroxide, magnesium carbonate, magnesium carbonate heavy, and magnesium sulfate.
(v) In any one of above (i) to (iv), there may be provided the oral solid formulation in which the sulfate includes at least one selected from the group consisting of sodium sulfate, potassium sulfate, and magnesium sulfate.
(vi) In any one of above (i) to (v), there may be provided the oral solid formulation in which the unit solid formulation is in the form of tablet, pellet, capsule, or pill.
(vii) In any one of above (i) to (vi), there may be provided the oral solid formulation which further includes a pharmaceutically acceptable additive.
(viii) In any one of above (i) to (vii), there may be provided the oral solid formulation in which the solid formulation includes about 70 to about 2000 unit solid formulations.
(ix) In any one of above (i) to (viii), there may be provided the oral solid formulation in which the solid formulation is for colon cleansing.
(x) In any one of above (i) to (ix), there may be provided the oral solid formulation in which the solid formulation includes the purgative component in a total amount of about 20 to about 500 g.
(xi) In any one of above (i) to (x), there may be provided the oral solid formulation in which the unit solid formulation includes the purgative component in an amount of about 20 mg to about 500 mg.

In the above, the solid formulations according to the embodiments of the present invention are exemplarily described, but the present invention is not limited thereto, and all combinations of various elements for the solid formulation according to the present invention described in the solid formulations of the present invention fall within the scope of the present invention.

The present invention may also provide a colon cleansing method including administering (an effective amount of) the oral solid formulation of the present invention.

The present invention may also provide a use of the oral solid formulation of the present invention for colon cleansing.

In the above method and use, the oral solid formulation of the present invention may be the same as that described in the oral solid formulation of the present invention.

### Advantageous Effects

The oral solid formulation of the present invention can minimize side effects such as dysphagia (swallowing disorder), gastrointestinal disorders like nausea or sickness when taken, and have excellent dosing convenience and medication compliance. In addition, the solid formulation of the present invention can be rapidly disintegrated and dissolved, can reduce side effects such as hyperemia, hemorrhage, erosion, edema, and the like in the stomach, and quickly exhibit a pharmaceutical effect, thereby reducing the time to start and finish discharging stool after the solid formulation is taken. Furthermore, it can be possible to minimize residues in the gastrointestinal tract and exhibit an excellent colon cleansing effect.

### Brief Description of the Drawings

FIG. 1 is a graph showing a result of evaluating side effects of Experimental Example 4.
FIG. 2 is a graph showing a result of evaluating colon cleansing ability of Experimental Example 7.

### Mode for Invention

Hereinafter, the present invention will be described in detail through preferred Examples for better understanding of the present invention. However, the following Examples are provided only for the purpose of illustrating the present invention, and thus the present invention is not limited thereto.

### Experimental Example 1: Identification of disintegration time

Tablets were prepared in accordance with components and contents as shown in table 1 below. Specifically, 109.375 g of anhydrous sodium sulfate, 19.563 g of potassium sulfate, 10 g of anhydrous magnesium sulfate, and 4.663 g of copovidone were mixed, after which 1 mL of purified water was slowly added and mixed, and then the mixture was passed through 20 mesh, and 1.4 g of stearic acid was added, mixed, and tableted with a tablet press.

**[Table 1]**

| Component | Example 1 (mg) | Comparative Example 1 (mg) |
|---|---|---|
| Anhydrous sodium sulfate | 109.375 | 1093.75 |
| Potassium sulfate | 19.563 | 195.63 |
| Anhydrous magnesium sulfate | 10.000 | 100.00 |
| Copovidone | 4.663 | 46.63 |
| Stearic acid | 1.400 | 14.00 |
| 1 tablet weight (mg) | 145.0 | 1450.0 |

A disintegration time of the tablets of Example 1 and Comparative Example 1 prepared above was measured in water according to the Disintegration Test of the Korean Pharmacopeia, General Tests.

The disintegration time of the tablets of Example 1 and Comparative Example 1 was 7 minutes 20 seconds and 17 minutes 30 seconds, respectively, and it was confirmed that the tablet of Example 1 is disintegrated much faster. It was confirmed that the tablet of Example 1, which is rapidly disintegrated, is more suitable as a solid colon cleansing agent.

### Experimental Example 2: Identification of dissolution time

Tablets were prepared in accordance with components and contents as shown in table 2 below. Specifically, 109.375 g of anhydrous sodium sulfate, 19.563 g of potassium sulfate, 10 g of anhydrous magnesium sulfate, and 5.062 g of copovidone were mixed, after which 3 mL of purified water was slowly added and mixed, and then the mixture was passed through 20 mesh, and 1.5 g of stearic acid was added, mixed, and tableted with a tablet press. After that, polyethylene glycol-polyvinyl alcohol copolymer was dissolved in purified water at a concentration of 5%, and then subjected to film coating with a coating machine.

**[Table 2]**

| Component | Example 2 (mg) | Comparative Example 2 (mg) |
|---|---|---|
| Anhydrous sodium sulfate | 109.375 | 1093.75 |
| Potassium sulfate | 19.563 | 195.63 |
| Anhydrous magnesium sulfate | 10.00 | 100.00 |
| Copovidone | 5.062 | 50.62 |
| Stearic acid | 1.50 | 15.00 |
| Polyethylene glycol-polyvinyl alcohol copolymer | 3.00 | 30.00 |
| 1 tablet weight (mg) | 148.50 | 1485.00 |

The dissolution time of 30 tablets of Example 2 and three tablets of Comparative Example 2 prepared was measured by the paddle method at about 37°C, 50 rpm, and 900 ml of pH 1.2 dissolution medium (dissolution tester, Pharmatest). Tablets of Example 2 and Comparative Example 2 were evaluated by putting ten tablets and one tablet in one vessel of the dissolution tester respectively. A dissolution rate was analyzed by liquid chromatography (HPLC).

It was confirmed that all of the tablets of Example 2 are dissolved within 15 minutes, whereas all of the tablets of Comparative Example 2 are eluted at about 30 minutes. It may be seen that the tablet of Example 2 is much more preferable as a solid colon cleansing agent compared to the tablet of Comparative Example 2.

### Experimental Example 3: Preparation and disintegration time of solid formulation

Tablets of Example 3 and Comparative Example 3 were prepared in accordance with the components and contents of table 3 below. Specifically, 123.15 g of polyethylene glycol 3350, 5.79 g of ascorbic acid, 7.27 g of sodium ascorbate, 9.24 g of anhydrous sodium sulfate, 3.31 g of sodium chloride, and 1.24 g of potassium chloride were passed through 20 mesh, mixed, and tableted with a tablet press to prepare a tablet.

**[Table 3]**

| Component | Example 3 (mg) | Comparative Example 3 (mg) |
|---|---|---|
| Polyethylene glycol 3350 | 123.15 | 1231.5 |
| Ascorbic acid | 5.79 | 57.9 |
| Sodium ascorbate | 7.27 | 72.7 |
| Anhydrous sodium sulfate | 9.24 | 92.4 |
| Sodium chloride | 3.31 | 33.1 |
| Potassium chloride | 1.24 | 12.4 |
| 1 tablet weight (mg) | 150 | 1500 |

A disintegration time of the tablets of Example 3 and Comparative Example 3 prepared above was measured in water according to the Disintegration Test method of the Korean Pharmacopeia, General Tests.

The disintegration time of the tablets of Example 3 and Comparative Example 3 was 5 minutes 45 seconds and 15 minutes 50 seconds, respectively, and it was confirmed that the tablet of Example 3 is disintegrated much faster. It was confirmed that the tablet of Example 3, which is rapidly disintegrated, is more suitable as a solid colon cleansing agent.

### Experimental Example 4: Assessment of side effects (1)

In order to confirm the amelioration of side effects of the solid formulation of the present invention, tablets were prepared in accordance with the components and contents of table 4 below. Specifically, 109.375 g of anhydrous sodium sulfate, 19.563 g of potassium sulfate, 10 g of anhydrous magnesium sulfate, and 5.062 g of copovidone were mixed, after which 3 mL of purified water was slowly added and mixed, and then the mixture was passed through 20 mesh, and 1.5 g of stearic acid was added, mixed, and tableted with a tablet press. After that, polyethylene glycol-polyvinyl alcohol copolymer was dissolved in 50% ethanol solution at a concentration of 10%, and then subjected to film coating with a coating machine.

**[Table 4]**

| Component | Tablet 1 (mg) | Tablet 2 (mg) | Tablet 3 (mg) |
|---|---|---|---|
| Anhydrous sodium sulfate | 109.375 | 218.75 | 1093.75 |
| Potassium sulfate | 19.563 | 39.125 | 195.625 |
| Anhydrous magnesium sulfate | 10.00 | 20.00 | 100.00 |
| Copovidone | 5.062 | 10.125 | 50.625 |
| Stearic acid | 1.50 | 3.00 | 15.00 |
| Polyethylene glycol-polyvinyl alcohol copolymer | 3.00 | 6.00 | 30.00 |
| 1 tablet weight (mg) | 148.50 | 297.00 | 1485.00 |

Side effects occurring in the gastrointestinal tract were observed by administering the prepared tablets 1 to 3 to beagles (about 10 kg). 80 tablets of tablet 1, 40 tablets of tablet 2, and 8 tablets of tablet 3 were orally administered so that the same amount of sulfate could be administered, and the observation was made under anesthesia to prevent vomiting. Each of tablets 1 to 3 was administered to three beagles together with water, and then euthanized at three hours to collect and incise the stomach, and the extent of damage to the gastric mucous membrane was evaluated with the naked eye with 0 to 16 points according to the area and intensity of occurrence of hyperemia, hemorrhage, erosion, and edema. Specifically, the extent of damage was evaluated as 0-4 points according to the area where side effects occurred and 0-4 points according to the intensity of side effects, and the two values were multiplied. The higher the point, the more the side effects.

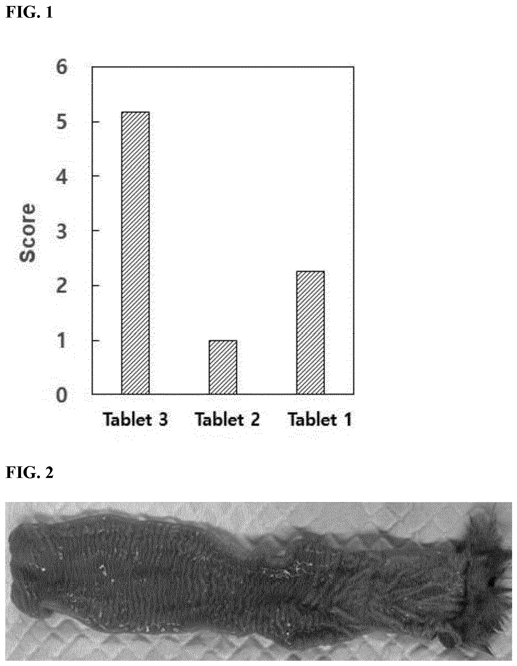

As a result of the evaluation of side effects, it can be found that the side effects in tablets 1 and 2 are much less than those in tablet 3 (FIG. 1). It is an unexpected result that tablets 1 and 2 have lower side effects than tablet 3 despite having a more number of tablets and a wider surface area in contact with the stomach.

Thus, it may be confirmed that tablets 1 and 2 according to the present invention may ameliorate side effects.

### Experimental Example 5: Assessment of side effects (2)

Tablets 1 to 3 of above table 4 were administered to evaluate the occurrence of side effects (edema) in the gastrointestinal tract through a tissue test. Tablets 1 to 3 were prepared and administered to three beagles, respectively, and a series of processes of cutting the stomach were performed in the same manner as in Experimental Example 4. The tissue was taken from the incised stomach to prepare a specimen for tissue test, and then stained with Hematoxylin & Eosin (H&E) so as to observe the occurrence of edema by using an optical microscope.

As a result, it was found that all individuals dosed with tablets 1 and 2 do not develop edema, whereas individuals dosed with tablet 3 develop edema (Table 5). It is an unexpected result that tablets 1 and 2 do not develop edema compared to tablet 3 despite having a more number of tablets and a wider surface area in contact with the stomach.

**[Table 5]**

| | Tablet 1 | Tablet 2 | Tablet 3 |
|---|---|---|---|
| Ratio of individuals with edema (%) | 0 | 0 | 100 |

Thus, it may be confirmed that tablets 1 and 2 according to the present invention may ameliorate side effects.

### Experimental Example 6: Evaluation of dosing convenience

Tablets 1 and 3 of above table 4 were administered to beagles to compare the dosing convenience. 80 tablets of tablet 1 and 8 tablets of tablet 3 were administered to beagles (about 10 kg) without anesthesia, so as to observe if the administration is performed without problems such as swallowing disorder, etc.

Tablet 3 is not easy to swallow, and thus may be administered only when an investigator forcibly pushes the same into a throat area by hand, whereas in the case of tablet 1, it was observed that several tablets are easily swallowed by the beagle per se at once, even though the number of administrations is ten times more than that of tablet 3. Accordingly, it can be seen that tablet 1 of the present invention is much more easily taken than tablet 3 having a weight similar to that of a commercial product, though the number of tablets to be taken is ten times more than that of tablet 3.

### Experimental Example 7: Evaluation of colon cleansing ability

60 tablets of tablet 1 prepared by the method of Experimental Example 4 were administered to a beagle (about 10 kg) without anesthesia, and euthanized three hours later, and then the large intestine was collected and excised to confirm colon cleansing ability.

As a result, as shown in FIG. 2, it could be seen that the large intestine is cleanly washed. Accordingly, it can be seen that the solid formulation of the present invention has excellent colon cleansing ability.

### Preparing Example 1: Preparation of solid formulation

Tablets were prepared in accordance with components and contents as shown in table 6 below. Specifically, in case of tablet 4, 138.938 g of anhydrous sodium sulfate, 4.663 g of copovidone, and 2.4 g of D-mannitol were mixed, after which 0.7 mL of purified water was slowly added and mixed, and then the mixture was passed through 20 mesh, and 1.0 g of stearic acid was added, mixed and tableted with a tablet press. After that, polyethylene glycol-polyvinyl alcohol copolymer was dissolved in 50% ethanol solution at a concentration of 10%, and then subjected to film coating with a coating machine. In case of tablet 5, 113.938 g of anhydrous sodium sulfate, 25.0 g of anhydrous magnesium sulfate, and 4.663 g of copovidone were mixed, after which 1.0 mL of purified water was slowly added and mixed, and then the mixture was passed through 20 mesh, and 1.4 g of stearic acid was added, mixed, and tableted with a tablet press. After that, polyethylene glycol-polyvinyl alcohol copolymer was dissolved in 50% ethanol solution at a concentration of 10%, and then subjected to film coating with a coating machine. In case of tablet 6, 113.938 g of anhydrous sodium sulfate, 25.0 g of potassium sulfate, 4.663 g of copovidone, and 2.4 g of D-mannitol were mixed, after which 0.7 mL of purified water was slowly added and mixed, and then the mixture was passed through 20 mesh, and 1.0 g of stearic acid was added, mixed and tableted with a tablet press. After that, polyethylene glycol-polyvinyl alcohol copolymer was dissolved in 50% ethanol solution at a concentration of 10%, and then subjected to film coating with a coating machine.

**[Table 6]**

| Component | Tablet 4 (mg) | Tablet 5 (mg) | Tablet 6 (mg) |
|---|---|---|---|
| Anhydrous sodium sulfate | 138.938 | 113.938 | 113.938 |
| Potassium sulfate | - | - | 25.0 |
| Anhydrous magnesium sulfate | - | 25.0 | - |
| D-mannitol | 2.4 | - | 2.4 |
| Copovidone | 4.663 | 4.663 | 4.663 |
| Stearic acid | 1.0 | 1.4 | 1.0 |
| Polyethylene glycol-polyvinyl alcohol copolymer | 3.0 | 3.0 | 3.0 |
| 1 tablet weight (mg) | 150.0 | 148.0 | 150.4 |

## Claims

1. An oral solid formulation comprising sulfate, wherein a unit solid formulation each of which has a weight of about 0.3 g or less.

2. The oral solid formulation of claim 1, wherein the sulfate is comprised in an amount of about 25 to about 60 g.

3. The oral solid formulation of claim 1, wherein the sulfate comprises at least one selected from the group consisting of sodium sulfate, potassium sulfate and magnesium sulfate.

4. The oral solid formulation of claim 3, wherein the sulfate comprises sodium sulfate and potassium sulfate.

5. The oral solid formulation of claim 3, wherein the sulfate comprises sodium sulfate, magnesium sulfate and potassium sulfate.

6. The oral solid formulation of claim 1, wherein simethicone is further comprised.

7. The oral solid formulation of claim 1, wherein the solid formulation comprises about 80 to about 1200 unit solid formulations.

8. An oral solid formulation comprising a purgative component, wherein a unit solid formulation each of which has a weight of about 0.5 g or less.

9. The oral solid formulation of claim 8, wherein the purgative component comprises at least one selected from the group consisting of polyethylene glycol, ascorbic acid, a salt of ascorbic acid, sorbitol, mannitol, citric acid, a salt of citric acid, a magnesium salt, carboxymethyl cellulose, a salt of carboxymethyl cellulose, lactulose, and sulfate.

10. The oral solid formulation of claim 9, wherein the magnesium salt comprises at least one selected from the group consisting of magnesium oxide, magnesium oxide heavy, magnesium hydroxide, magnesium carbonate, magnesium carbonate heavy, and magnesium sulfate.

11. The oral solid formulation of claim 9, wherein the sulfate comprises at least one selected from the group consisting of sodium sulfate, potassium sulfate and magnesium sulfate.

12. The oral solid formulation of claim 8, wherein the solid formulation comprises about 70 to about 2000 unit solid formulations.

13. The oral solid formulation of claim 1 or 8, wherein each unit solid formulation has a weight of about 0.05 g or more.

14. The oral solid formulation of claim 1 or 8, wherein a pharmaceutically acceptable additive is further comprised.

15. The oral solid formulation of claim 1 or 8, wherein the unit solid formulation is in a form of tablet, pellet, capsule, or pill.

16. The oral solid formulation of claim 1 or 8, wherein the solid formulation is for colon cleansing.
